Europäisches Patentamt

European Patent Office

Office européen des brevets

(11) Veröffentlichungsnummer: **0 159 585**
**B1**

## EUROPÄISCHE PATENTSCHRIFT

(45) Veröffentlichungstag der Patentschrift: **14.03.90**

(21) Anmeldenummer: **85103975.0**

(22) Anmeldetag: **02.04.85**

(51) Int. Cl.⁵: **C 12 P 7/56,** C 07 C 51/48, C 07 C 59/08

(54) **Verfahren zur Isolierung von Milchsäure aus feststoffhaltigen wässrigen Lösungen.**

(30) Priorität: **21.04.84 DE 3415141**

(43) Veröffentlichungstag der Anmeldung:
**30.10.85 Patentblatt 85/44**

(45) Bekanntmachung des Hinweises auf die
Patenterteilung:
**14.03.90 Patentblatt 90/11**

(84) Benannte Vertragsstaaten:
**BE CH DE FR GB LI NL**

(56) Entgegenhaltungen:
**DE-A-3 222 837**
**US-A-2 420 234**
**US-A-2 712 516**

(73) Patentinhaber: **BASF Aktiengesellschaft**
**Carl-Bosch-Strasse 38**
**D-6700 Ludwigshafen (DE)**

(72) Erfinder: **Berning, Wilfried, Dr.**
**Klosterweg 8**
**D-6520 Worms 1 (DE)**
Erfinder: **Siegel, Hardo, Dr.**
**Hans-Purrmann-Allee 25**
**D-6720 Speyer (DE)**
Erfinder: **Heida, Bernd**
**Dresdener Strasse 11**
**D-6737 Boehl-Iggelheim (DE)**
Erfinder: **Wickenhaeuser, Gerhard, Dr.**
**Sanderstrasse 89 b**
**D-5060 Bergisch-Gladbach 2 (DE)**

**Beschreibung**

Die Erfindung betrifft ein Verfahren zur Isolierung von Milchsäure aus feststoffhaltigen wäßrigen Lösungen durch Extraktion mit organischen Lösungsmitteln.

Bekanntlich stellt man L(−) Milchsäure großtechnisch durch fermentativen Abbau glucosehaltiger Rohstoffe her (Ind. Eng. Chem. 44, 1958 (1952)). Die Herstellung von D-Milchsäure erfolgt durch geeignete Bakterien in ähnlicher Weise. Die Fermentation führt man in Gegenwart von Calciumcarbonat durch, so daß man eine wäßrige Lösung oder Suspension erhält, die 6 bis 20 Gew.% Calciumlactat enthält. Anschließend wird diese Lösung mit Schwefelsäure angesäuert, wobei Gips ausfällt, den man zusammen mit der Bakterienmasse abfiltriert. Als Filtrat erhält man eine wäßrige Milchsäurelösung, die in der Regel aufkonzentriert und geeigneten Reinigungsoperationen unterworfen wird.

Das Aufkonzentrieren der verdünnten wäßrigen Milchsäurelösung bereitet häufig Schwierigkeiten, da Reste der Bakterienmasse sowie des verwendeten Nährmediums und gelöstes Calciumsulfat in den Apparaturen zu Verkrustungen führen. Aus diesem Grunde wurde bereits mehrfach der Vorschlag gemacht, die Milchsäure aus den wäßrigen Lösungen nach Entfernung der Feststoffe durch Filtration mit Hilfe von Lösungsmittel, wie Alkoholen, Ether, Ketone und Ester zu extrahieren (z.B. DE—OS 12 68 088 und DE—OS 32 22 837).

Da sich jedoch die Filtration, insbesondere bedingt durch die feinverteilte Biomasse, als eine äußerst aufwendige, kostenintensive und verlustreiche Arbeitstechnik erwiesen hat, wurde versucht, die Feststoffabtrennung durch Extraktion der unfiltrierten, feststoffhaltigen Milchsäurelösung zu umgehen. Dabei mußte jedoch festgestellt werden, daß sich der Feststoff und vor allem die Biomasse nach Zusatz verschiedener Lösungsmittel auf beide Phasen verteilt. Selbst nach sehr langer Absitzzeit blieb ein erheblicher Teil des Feststoffs in der phasengrenze und mußte abfiltriert werden.

Es wurde nun gefunden, daß man Milchsäure aus wäßrigen Lösungen durch kontinuierliche Extraktion mit einem organischen Lösungsmittel unter Vermeidung der genannten Schwierigkeiten isolieren kann, wenn man eine durch Ansäuern einer fermentativ hergestellten wäßrigen Lösung oder Suspension von Calciumlactat mit Schwefelsäure erhaltene Suspension, die als Feststoffe Gips und Biomasse enthält, in einer Kolonne, die mit solchen Einbauten ausgerüstet ist, welche aus hydrophobem Material bestehen oder mit hydrophobem Material beschichtet sind im Gegenstrom mit dem Lösungsmittel in Berührung bringt, und die Milchsäure aus der ablaufenden Lösungsmittelphase gewinnt.

Nach dem neuen Verfahren gelingt die Isolierung der Milchsäure aus den unfiltrierten Suspensionen glatt und auf technisch besonders einfache Weise. Das ist sehr überraschend, weil die Extraktion z.B. nur in einer Siebbodenkolonne gelingt, in der die Einbauten hydrophob sind.

Als Ausgangslösungen werden technische Milchsäurelösungen verwendet, die auf an sich bekannte Weise durch Ansäuern einer fermentativ hergestellten wäßrigen Lösung von Calciumlactat mit Schwefelsäure erhalten werden. Bei diesen Lösungen handelt es sich um Suspensionen, die als Feststoffe insbesondere Gips und Biomasse enthalten. Unter Biomasse wird die Bakterienmasse zusammen mit Resten des verwendeten Nährmediums, wie Hefen, verstanden. Suspensionen dieser Art haben z.B. die folgenden Zusammensetzung: 5 bis 15 Gew.% Milchsäure, 5 bis 20 Gew.% Gips, 0,1 bis 2 Gew.% Schwefelsäure, 0,5 bis 5 Gew.% Biomasse; der Rest ist überwiegend Wasser.

Nach dem erfindungsgemäßen Verfahren wird die wäßrige Suspension in einer mit hydrophoben Einbauten ausgerüsteten Kolonne im Gegenstrom mit dem Lösungsmittel in Berührung gebracht. Als Kolonnen kommen z.B. alle bekannten Kolonnentypen in Betracht, soweit sie von der Konstruktion her feststoffverträglich sind. Die Kolonnen müssen mit hydrophoben Einbauten, wie solchen aus Teflon oder kunststoffbeschichtetem Stahl, ausgerüstet sein. Besonders zweckmäßig sind pulsierte Siebbodenkolonnen und Füllkörperkolonnen mit geordneten Füllkörpern. Die wäßrige Suspension wird dem oberen Teil der Kolonne zugeführt und als disperse Phase geführt. Das Lösungsmittel wird zur dispersen wäßrigen Phase im Gegenstrom geführt. Nach der Extraktion der wäßrigen Suspension mit dem Lösungsmittel, die man bei Temperaturen von 10 bis 90°C durchführt, entnimmt man der Kolonne eine wäßrige sowie eine organische Phase. Die Milchsäure wird dann auf an sich bekannte Weise aus der Lösungsmittelphase abgetrennt.

Als Lösungsmittel sind organische Lösungsmittel geeignet, die sich bei den Temperaturen der Extraktion nicht beliebig mit Wasser mischen und die einen Verteilungskoeffizienten von > 0,1 aufweisen, wobei der Verteilungskoeffizient das Verhältnis des Milchsäuregehaltes zwischen der Lösungsmittelphase und der wäßrigen Phase im Gleichgewichtszustand bedeutet. Lösungsmittel der genannten Art sind z.B. Ether, wie Diisobutylether, Ester, wie Isobutyllactat oder Pentyllactat, Ketone, wie Butanon oder Methylethylketon und Alkohole, wie Alkanole mit mindestens 4 C-Atomen, z.B. Butanol, iso-Butanol, Pentanol, Methylbutanol. Neben den reinen Lösungsmitteln lassen sich auch Lösungsmittelgemische einsetzen. Die Lösungsmittelmenge ist vom jeweiligen Verteilungskoeffizienten abhängig. Sie kann die 0,1 bis 10-fache Gewichtsmenge des wäßrigen Ausgangsgemisches betragen. Vorzugsweise nimmt man die 0,5 bis 3-fache Gewichtsmenge Lösungsmittel, bezogen auf Ausgangsgemisch. Die Verwendung der Alkohole als Lösungsmittel hat zwei Vorteile. Einerseits sind die Verteilungskoeffizienten stark von der Extraktionstemperatur abhängig. Bei i-Butanol ist z.B. eine Halbierung der Lösungsmittelmenge bei einer Erhöhung der Extraktionstemperatur von 20 auf 70°C möglich. Andererseits wird bevorzugt bei erhöhter Temperatur die Milchsäure in der Extraktionskolonne partiell in die entsprechenden Ester überführt, was zu einer weiteren Lösungsmitteleinsparung und zu einer Verringerung des Wassergehaltes im Extrakt führt. Aufgrund der erwähnten Vorteile ergibt sich eine Einsparung an Destillationskosten von > 50%.

Besonders vorteilhaft ist ein Gehalt von überschüssiger Schwefelsäure in der eingesetzten wäßrigen Suspension, da diese teilweise zusammen mit der Milchsäure extrahiert wird und bei der Veresterung als katalysator dient. Die vollständige Umsetzung kann in bekannter Weise in einer nachfolgenden diskontinuierlichen oder kontinuierlichen Veresterungsstufe erfolgen, beispielsweise durch Erhitzen der Lösungsmittelphase auf Temperaturen von 60 bis 140°C und Abdestillieren des Reaktionswassers, gegebenenfalls unter Vakuum. Die durch Destillation in reiner Form erhaltenen Milchsäureester sind wertvolle Zwischenprodukte. Sie können auch in bekannter Weise wieder zu Milchsäure und Alkohol zurückgespalten wetden, so daß auf diese Weise hochreine Milchsäure erhalten wird.

Beispiel

Auf den Kopf einer pulsierenden, mit Siebböden aus Teflon ausgerüsteten Kolonne wird kontinuierlich pro Stunde eine durch Fermentation erhaltene wäßrige Milchsäurelösung, die 413 Gew.-Teile Milchsäure, 3 085 Gew.-Teile Wasser, 52 Gew.-Teile Schwefelsäure und als Feststoffe 505 Gew.-Teile Gips und Biomasse enthält, als disperse Phase gegeben. Zur Extraktion leitet man gleichzeitig im Gegenstrom zu der wäßrigen dispersen Phase bei 70°C 4 600 Gew.-Teile mit Wasser gesättigtes Isobutanol von unten nach oben durch die Kolonne. Aus dem Gemisch erhält man durch Phasentrennung eine feststofffreie Isobutanolphase und eine wäßrige feststoffhaltige Phase. Die Isobutanolphase, die man kontinuierlich oberhalb des Zulaufs der wäßrigen Lösung aus der Kolonne abzieht, enthält > 97% der eingesetzten Milchsäure. Ca. 25% der extrahierten Milchsäure fallen in Form des Milchsäureisobutylesters an. Die in der Isobutanolphase gelöste Milchsäure wird anschließend durch Abdestillieren des Reaktionswassers bei 60 bis 140°C vollständig in den Isobutylester übergeführt, den man im Vakuum destilliert (Sdp. 80°C bei 25 mbar). Die Ausbeute, bezogen auf Milchsäure, liegt bei 95%.

## Patentansprüche

1. Verfahren zur Isolierung von Milchsäure aus einer wäßrigen Lösung durch kontinuierliche Extraktion mit einem organischen Lösungsmittel, dadurch gekennzeichnet, daß man eine durch Ansäuern einer fermentativ hergestellten wäßrigen Lösung oder Suspension Von Calciumlactat mit Schwefelsäure erhaltene Suspension, die als Feststoffe Gips und Biomasse enthält, in einer Kolonne, die mit solchen Einbauten ausgerüstet ist, welche aus hydrophobem Material bestehen oder mit hydrophobem Material beschichtet sind, im Gegenstrom mit dem Lösungsmittel in Berührung bringt, und die Milchsäure aus der ablaufenden Lösungsmittelphase gewinnt.

2. Verfahren nach Anspruch 1, dadurch gekennzeichnet, daß man als Lösungsmittel Butanole oder Pentanole verwendet.

3. Verfahren nach Anspruch 1, dadurch gekennzeichnet, daß man die in der Kolonne bei der Extraktion mit Butanolen oder Pentanolen erhältliche Lösungsmittelphase zur Vervollständigung der Milchsäureesterbildung unter Abdestillieren von Wasser erhitzt.

## Revendications

1. Procédé d'isolement de l'acide lactique à partir d'une solution aqueuse par extraction continue avec un solvant organique, caractérisé en ce qu'on met en contact, à contre-courant avec le solvant, une suspension obtenue par acidification par l'acide sulfurique d'une solution ou suspension aqueuse de lactate de calcium obtenue par fermentation qui contient comme matières solides du gypse et une biomasse, dans une colonne qui est garnie d'éléments similaires qui sont en matière hydrophobe ou sont revêtus d'une matière hydrophobe, et on récupère l'acide lactique de la phase sol'vant qui s'écoule.

2. Procédé selon la revendication 1, caractérisé en ce qu'on utilise comme solvant des butanols ou des pentanols.

3. Procédé selon la revendication 1, caractérisé en ce qu'on chauffe la phase solvant obtenue dans la colonne par extraction avec des butanols ou des pentanols pour compléter la formation d'esters d'acide lactique avec élimination de l'eau par distillation.

## Claims

1. A process for isolating lactic acid from an aqueous solution by continuous extraction with an organic solvent, which comprises contacting a suspension obtained by acidulating a fermentatively prepared aqueous solution or suspension of calcium lactate with sulfuric acid, this suspension containing gypsum and biomass as solids, in a column equipped with internal fitments consisting of or coated with hydrophobic material in countercurrent with the solvent and isolating the lactic acid from the solvent phase runoff.

2. A process as claimed in claim 1, wherein the solvent used is a butanol or a pentanol.

3. A process as claimed in claim 1, wherein the solvent phase obtainable in the column on extraction with a butanol or a pentanol is heated, with distillative removal of water, to complete the lactic ester formation.